# EUROPÄISCHE PATENTSCHRIFT

(11) **EP 3 266 408 B1**
(45) Veröffentlichungstag und Bekanntmachung des Hinweises auf die Patenterteilung: **10.06.2020**
(21) Anmeldenummer: 16178660.3
(22) Anmeldetag: 08.07.2016
(51) Int. Cl.: A61C 9/00, A61C 13/00

(54) **ABFORMLÖFFEL, ABFORMANORDNUNG SOWIE VERFAHREN ZUR HERSTELLUNG EINER PROTHESE**
IMPRESSION TRAY, IMPRESSION ASSEMBLY AND METHOD OF MANUFACTURING A PROSTHESIS
PORTE-EMPREINTE, SYSTEME D'EMPREINTE ET PROCEDE DE FABRICATION D'UNE PROTHESE

(43) Veröffentlichungstag der Anmeldung: 10.01.2018
(60) Teilanmeldung: 19182870.6
(73) Patentinhaber: Ivoclar Vivadent AG, 9494 Schaan (LI)
(72) Erfinder: Watzke, Ronny, 6800 Feldkirch (AT); Frei, Christian, 39025 Naturns (IT)
(74) Vertreter: Baronetzky, Klaus

(56) Entgegenhaltungen:
- WO-A1-2015/089676
- DE-A1-102012 111 020
- DE-A1-102013 102 421
- DE-T2- 69 935 969
- US-A- 5 961 325
- US-A1- 2009 117 514
- US-A1- 2016 008 106

## Beschreibung

Die Erfindung betrifft einen Abformlöffel nach dem Oberbegriff von Anspruch 1, eine Abformanordnung gemäß Anspruch 9, sowie ein Verfahren nach Anspruch 11.

Es ist seit langem bekanntgeworden, für die Erfassung der Mundsituation eines Patienten sogenannte Abformlöffel zu verwenden. Hier haben sich verschiedene Systeme etabliert. Abgesehen von der einfachen und statischen Abformung beispielsweise eines Kieferkammes ist in vielen Fällen auch der Bezug zum gegenüberliegenden Kiefer relevant. Hierzu ist es bekanntgeworden, entsprechend geeignete Abformlöffel für Oberkiefer und Unterkiefer je getrennt vorzunehmen und die Relativposition über Registrierstifte, über die sogenannte Bissregistrierung, vorzunehmen.

Ferner ist es bekannt, an die Kieferkammbögen jeweils Wachswälle anzuschließen, um so die Relativposition von Oberkiefer und Unterkiefer zu erfassen, beziehungsweise festzulegen.

Ein Beispiel für eine derartige Lösung ist aus der DE 20 218 063 U1 ersichtlich.

Das Dokument DE 699 35 969 T2 offenbart ein Abformlöffel nach dem Oberbegriff des Anspruchs 1.

Herr Josephus Schreinemaker brachte vor längerer Zeit Abformlöffel auf den Markt, deren Besonderheit in der Autoklavierbarkeit liegt. So sind Abformlöffel bekanntgeworden, die aus einer mit Durchbrechungen versehenen Metallbasis bestehen, auf welcher Metallbasis die Abformmasse aufgebracht ist. Hierzu ist eine Trägerplatte des Abformlöffels metallisch und daher temperaturbeständig beispielsweise bei 120 oder 140 Grad Celsius. Über Durchbrechungen lässt sich das eingebrachte Abformmaterial, dass beispielsweise wachsartig sein kann, in dem Äbformlöffel verankern.

Wenn eine Funktionsabformung erwünscht ist, muss der Abformlöffel geführt werden, so dass ein Löffelgriff vorgesehen ist, der durch den Mund hindurch verläuft. Dies wird vom Patienten teilweise als unangenehm empfunden; zudem hängt die Qualität der Abformung stark von der Person ab, die die Abformung vornimmt. Bei einem Löffelgriff wird die Funktionsabformung aufgrund des mundoffenen Verfahrens und durch die Finger des Behandlers mitunter negativ beeinflusst.

Demgegenüber liegt der Erfindung die Aufgabe zugrunde, einen Abformlöffel gemäß dem Anspruch 1, eine Abformanordnung gemäß dem Anspruch 9 und ein Verfahren gemäß dem Anspruch 11 zu schaffen, die für die Bereitstellung einer besser angepassten Prothese geeignet sind, und zugleich eine bessere Akzeptanz beim Patienten ermöglicht.

Diese Aufgabe wird erfindungsgemäß durch Anspruch 1, bzw. 9 und 11 gelöst. Vorteilhafte Weiterbildungen ergeben sich aus den Unteransprüchen.

Erfindungsgemäß ist es vorgesehen, dass die Abformlöffel aus den zugehörigen Kieferkammbogen und Wachswällen bestehen, die zueinander einstückig ausgebildet sind.

Bei Bedarf lässt sich der so realisierte Abformlöffel dann in seiner Form anpassen. Der Abformlöffel ist bei einer Temperatur von deutlich mehr als der Mundtemperatur verformbar, beispielsweise 60,80 oder unter 100 Grad Celcius, und zwar mit einer Norm-Handkraft, die weniger als 100 N oder weniger als 50 N beträgt.

Ein besonderer Vorteil des Kombination des erfindungsgemäßen Abformlöffels mit einem Referenzmuster, das im Frontbereich auf diesem aufgebracht ist, ist, dass die Behandlung des Patienten auf 2 Termine reduzierbar ist: Zum einen die Abformung und Datenerfassung, zum anderen das Einsetzen der neuen Prothesen. Aufgrund der besseren Anpassbarkeit müssen auch für unterschiedliche Größen weniger verschiedene Abformlöffel bereitgehalten werden, beispielsweise lediglich eine kleine, Aufgrund der besseren Anpassbarkeit müssen auch für unterschiedliche Größen weniger verschiedene Abformlöffel bereitgehalten werden, beispielsweise lediglich eine kleine, eine mittlere und eine große Größe, so dass die Erstauswahl des betreffenden Abformlöffels oder Wachslöffels auch ohne Vermessung auf Sicht erfolgen kann.

Die Verformung kann beispielsweise so erfolgen, dass der erfindungsgemäße Abformlöffel in ein warmes Wasserbad gelegt wird, das eine Erwärmung auf beispielsweise 50 oder 60 Grad und ein gegebenenfalls erforderlichen Verformen extraoral ermöglicht.

Die Verformung erfolgt bevorzugt in bukkal-oraler Richtung, wobei es sich versteht, dass auch in Anpassung an die Patientenanatomie eine partielle Vertikalverformung, also in okklusal-gingivaler Richtung, für jede Seite des Kieferkammbogens unabhängig von der anderen möglich ist.

Die insofern vorbereiteten Kieferkammbögen mit integrierten Wachswällen werden nun intraoral überprüft, und es wird an den Okklusionsseiten der Bisswälle festgestellt, ob das Aufeinanderliegen dieser spaltfrei möglich ist. Wenn ein Spalt sichtbar ist, werden überschüssige Bereiche mit einem hierzu geeigneten Werkzeug, beispielsweise einem sogenannten Rimformer, abgetragen, bis beide Okklusionsseiten der Wachswälle plan aufeinander aufliegen.

Die Verformung, wie sie hier erläutert wird, bezieht sich auf die plastische Verformbarkeit. Es versteht sich, dass je nach Materialwahl zusätzlich zur plastischen Verformbarkeit auch eine elastische Mikroverformbarkeit vorliegt. Der erfindungsgemäße Abformlöffel federt in diesem Fall beim Verformen ganz leicht zurück, wobei es bevorzugt ist, die elastische Verformbarkeit sehr gering zu halten, zumindest im Vergleich zur tatsächlich erfolgten plastischen Verformung.

Das genannte Verhältnis wird wesentlich von der Materialwahl, aber auch der Verformungstemperatur bestimmt. Wenn beispielsweise eine typisches Dentalwachs auf hohe Temperaturen wie 80 oder 85 Grad erwärmt wird, ist die elastische Verformbarkeit nahezu entfallen. Bei diesen Temperaturen ist allerdings die Formtreue nicht in allen Umständen gewährleistet, so dass es bevorzugt ist, die Verformungstemperatur so zu wählen, dass die elastische Verformbarkeit nicht mehr merkbar besteht, jedoch die Formtreue erhalten bleibt.

Nach der planen Ausrichtung der Wachswälle erfolgt die Funktionsabformung bei geschlossenem Mund. Die beiden Abformlöffel oder Kieferkammbögen mit integrierten Wachswällen werden bei geschlossenem Mund durch einen entsprechenden durch den Patienten auszuübenden Druck aufeinander gedrückt, nachdem sie mit einem geeigneten Abformmaterial gefüllt worden sind.

Das Abformmaterial wird hierdurch verformt.

Hieran anschließend wird bevorzugt ein Okklusionom, also ein Gesichtsbogen mit angeformter Bissgabel, so positioniert, dass die Bissgabel zwischen beiden Wachswällen zu liegen kommt. Die Okklusionsebene ist so definiert, und die so erfassten Werte werden notiert.

Besonders bevorzugt ist es, wenn der Oberkiefer-Wachswall an seinem distalen Ende eine Abschrägung aufweist.

Hieran anschließend können bei geöffneten Mund des Patienten bei Bedarf Ästhetiklinien, also mindestens Lippenschlusslinien und Mittellinien, ggf. auch Lachlinien, im labialen Bereich eines Wachswalls, insbesondere des Oberkiefers-Wachswalls angezeichnet werden, und es werden dort Referenzmuster aufgebracht, die bei geöffneten Lippen, also beim Lächeln des Patienten, vollständig sichtbar sind.

Die Referenzmuster können in beliebiger geeigneter Weise ausgestaltet sein:
Es ist beispielsweise möglich, einen entsprechenden Aufkleber anzubringen; es kann eine entsprechende Wachsform aufgebracht werden, oder es kann über einen Stempel ein entsprechendes Wachsmuster in den Wachswall kalt oder heiß eingebracht werden.

Es ist auch möglich, dass das Referenzmuster bereits auf dem Kieferkammbogen mit integriertem Wachswall vorhanden ist.

Ferner besteht auch die Möglichkeit, dass das Referenzmuster auf einem aus Kunststoff gefrästem Abformlöffel bereits vorhanden ist.

Es wird nun ein 2-dimensionales Bild des Mundes des Patienten mit gespreizten Lippen erstellt, wobei die Referenzkörper des Referenzmusters sämtlich sichtbar sein sollen.

Für die hieran anschließende Bissregistrierung kann beispielsweise ein Verschlüsselungsmaterial zwischen die Okklusionsebenen der Wachswälle gebracht werden. Alternativ kann ein Stützstiftregistrat, beispielsweise das Gnathometer CAD der Fa. Wieland Dental, Pforzheim, in mindestens einen Wachswall heiß eingedrückt werden und so die Kieferkammbögen (20) gegeneinander verschlüsseln.

Die verschlüsselten Kieferkammbögen mit integrierten Wachswällen werden nun gemeinsam, also in verschlüsselter Form, von einer Scannvorrichtung 3-dimensional gescannt, und die Okklusionsebene, die auch basierend auf den Werten des Okklusionoms erfasst wurde, wird von einer Verarbeitungsvorrichtung in die Scandaten eingebracht.

Anschließend heran erfolgt durch die Verarbeitungsvorrichtung die virtuelle Zahnaufstellung, wobei die Zähne in an sich bekannter Weise nach zahntechnischen Regeln, gegebenenfalls unter Beachtung der aufgebrachten Ästhetiklinien, angeordnet werden.

Anschließend hieran wird das zuvor aufgenommene Bild des Referenzmusters mit dem um dieses herum sichtbaren Lippen des Patienten, das auch als Lächelbild bezeichnet wird, mit der virtuellen Zahnaufstellung zusammengeführt und in Übereinstimmung gebracht. Die Zusammenführung erfolgt zwischen dem 2D-Lächelbild und den 3D-Scandaten der Abformung einschließlich des gescannten Referenzmusters.Die Ausrichtung und die Höhenlage der Oberkiefer-Schneidezähne - und damit indirekt sämtlicher Zähe wird so perfekt entsprechend dem Lächelbild realisiert, was erhebliche ästhetische Vorteile gegenüber einer rein funktionsbezogenen Aufstellung bietet.

Die CAD-Daten werden basierend hierauf, also nach Anpassung der Lage der Zähne an das "Lächelbild", bereitgestellt, und die Zähne werden nun in geeigneter Weise gefertigt, beispielsweise gdurch auftragende oder abtragende Verfahren.hergestellt. Eine Anpassung erfolgt nun ggf., grundsätzlich werden die Zähne basierend auf den Ästhetiklinien aufgestellt.

Weitere Einzelheiten, Vorteile und Merkmale ergeben sich aus der Beschreibung der nachstehenden Ausführungsbeispiele der Erfindung anhand der Zeichnung.

Es zeigen:
- Fig. 1: eine Abbildung mehrerer Abformlöffel in erfindungsgemäßer Ausgestaltung, je bestehend aus einem Unterkiefer-Abformlöffel und einem Oberkiefer-Abformlöffel, und je in unterschiedlichen Größen;
- Fig. 2: eine perspektivische Darstellung einer vollständigen Anordnung eines Abformlöffels, der für die Aufnahme von Abdruckmasse bereit ist;
- Fig. 3: eine Darstellung eines Oberkiefer- und eines Unterkiefer-Abformlöffels, wobei unter Einsatz eines Rimformers ein klaffender Spalt vermieden wird;
- Fig. 4: eine Ausführungsform eines Oberkiefer-Abformlöffels mit aufgebrachtemAbformmaterial;
- Fig. 5: eine distale Ansicht einer verschlüsselten Abformlöffel-Anordnung mit aufgebrachter Abdruckmasse und genommenen Abdruck;
- Fig. 6: ein 2-dimensionales Bild eines geöffneten Mundes mit eingeblendetem Oberkiefer-Abformlöffel, soweit sichtbar, unter Darstellung eines Referenzmusters;
- Fig. 7: eine Frontansicht einer virtuellen Darstellung des Oberkiefer- und des Unterkiefer-Abformlöffels mit aufgebrachtem Referenzmuster;
- Fig. 8: eine perspektivische Darstellung eines Oberkiefer-Abformlöffels in einer weiteren Ausführungsform;
- Fig. 9: eine perspektivische Darstellung eines Unterkiefer-Abformlöffels in einer weiteren Ausführungsform; und
- Fig. 10: eine perspektivische Darstellung eines Oberkiefer- und eines Unterkiefer-Abformlöffels in einer weiteren Ausführungsform.

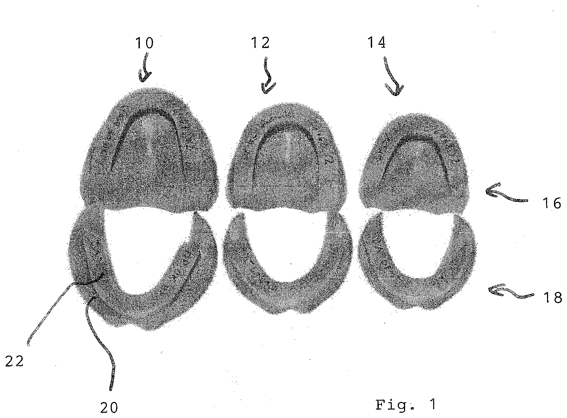

Aus Fig. 1 ist eine erfindungsgemäße mögliche Ausgestaltung von Abformlöffeln ersichtlich. Es handelt sich hier um Anordnungen mit einem großen Abformlöffel 10, einem mittleren 12 und einem kleinen 14. Diese bestehen je aus einem Oberkiefer-Abformlöffel 16 und einem Unterkiefer-Abformlöffel 18. Jeder Abformlöffel besteht aus einem Kieferkammbogen, der für die Aufnahme von Abformmasse für die Abdrucknahme bestimmt ist, und je einem Wachswall 22, wobei Kieferkammbogen 20 und Wachswall 22 einstückig aneinander angeformt sind.

Für die Unterkiefer-Abformbögen verlaufen die Wachswälle und Kieferkammbögen 22 und 20 je entsprechend dem Kieferkamm, also im wesentlichen U-förmig.

Für die Oberkiefer-Abformlöffel 16 ist zusätzlich ein Gaumenschild 24 vorgesehen, der sich zwischen den beiden Enden des betreffenden Oberkiefer-Kammbogens erstreckt, wie es an sich bekannt ist.

Die Abformlöffel bestehen aus einem Material mit einem Erweichungspunkt zwischen 40 und etwa 100 Grad Celsius. Für die Anpassung an den betreffenden Kieferkamm des Patienten lassen sie sich dementsprechend erhöhter Temperatur - beispielsweise nach Entnahme aus einem Warmwasserbad - in geeigneter Weise verformen.

Die Bereitstellung von drei Größen von Abformlöffeln ermöglicht es gepaart mit dieser Maßnahme, sämtliche in der Praxis vorkommenden Kieferformen von Patienten abzudecken.

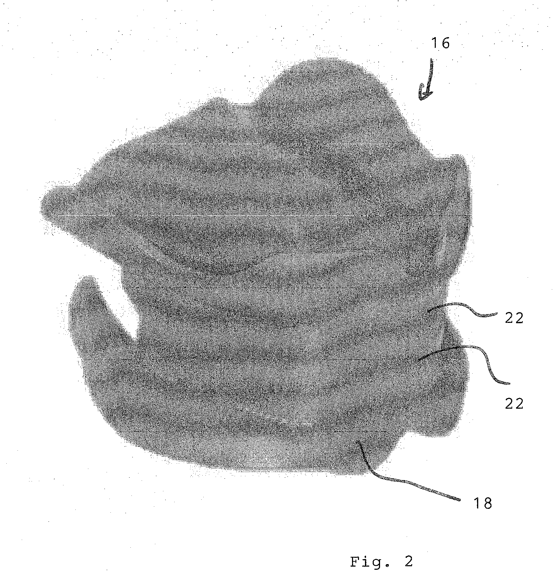

Aus Fig. 2 ist eine Anordnung aus einem Oberkiefer-Abformlöffel 16 und einem Unterkiefer-Abformlöffel 18 ersichtlich. In der dargestellten Ausgestaltung fluchten die Wachswälle miteinander und liegen ohne Spalt mit Ihren Okklusionsflächen aneinander an.

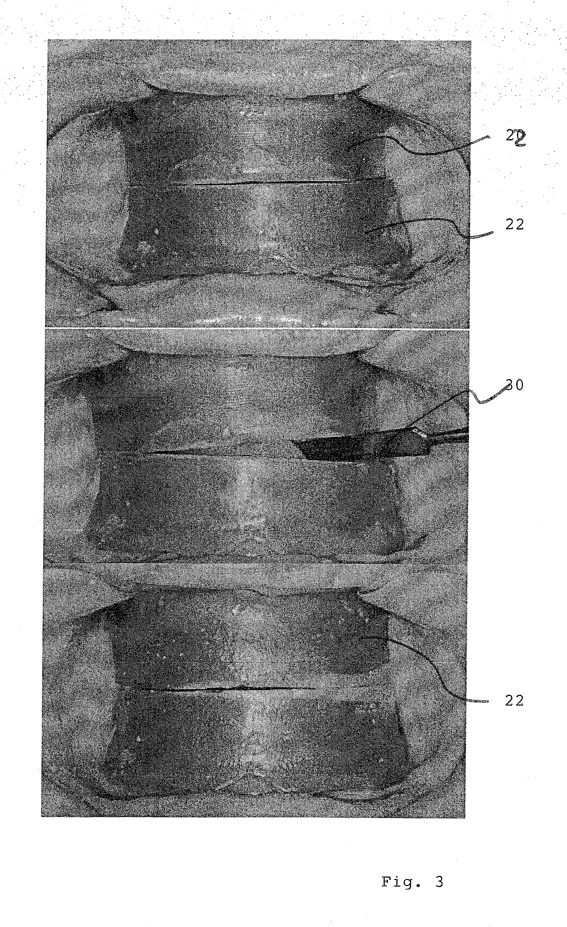

Fig. 3 zeigt Maßnahmen, die eine spaltfreie Okklusion der Wachswälle 22 miteinander ermöglichen. Wenn ein einseitiger Spalt 30 entsteht, wie es aus dem mittleren Bild aus Fig. 3 ersichtlich ist, lässt sich dieser über ein entsprechendes Werkzeug füllen, so dass wiederum eine im wesentlichen spaltfreier Anlage entsteht, wie es aus dem unteren Bild von Fig. 3 ersichtlich ist.

Alternativ lässt sich auch an der in der Darstellung linken Seite der Wachswälle Material abtragen.

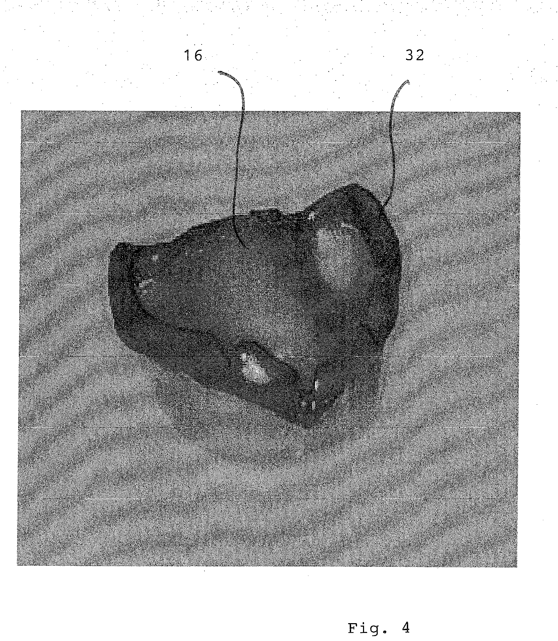

Aus Fig. 4 ist ein Abformlöffel 16 ersichtlich, dessen Kieferkamm-Nuten bereits mit Abformmaterial 32 gefüllt sind. Das Füllen kann in jeglicher geeigneter Weise erfolgen, wobei die Bereitstellung eines entsprechenden Abformmaterial-Überschuss es ermöglicht, dass sämtliche Einzelheiten der Kieferkämme abgeformt werden können.

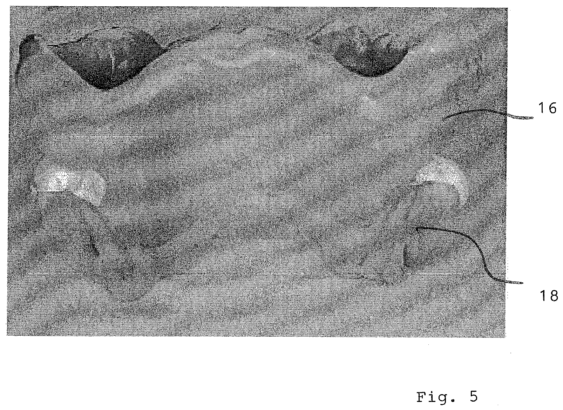

Eine distale Ansicht von Abformlöffeln 16 und 18 ist aus Fig. 5 zu entnehmen. Dort ist die Abformung bereits erfolgt und die verschlüsselten Abformlöffel sind aus dem Mund des Patienten entnommen, so dass sie gescannt werden können.

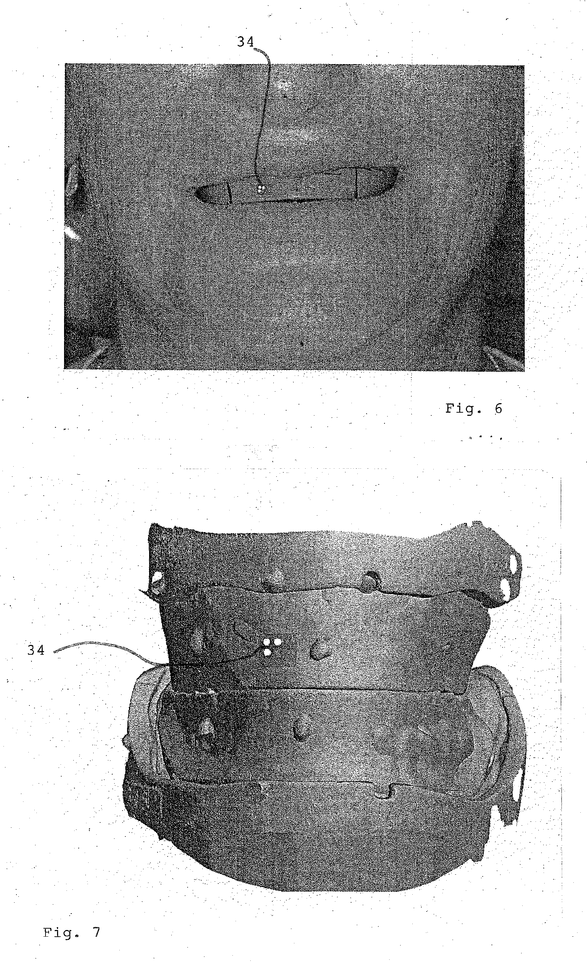

Aus Fig. 6 ist ein so genanntes Lächelbild einer Patientin bzw. eines Patienten ersichtlich. Der Patient öffnet die Lippen, so dass die Oberkieferzähne - oder bei einem Zahnlosen Patienten die Oberkiefer - sichtbar sind.

Der Zahnarzt bringt nun ein Referenzmuster 34 dort an. Ein entsprechendes Referenzmuster wird auf dem Oberkiefer-Wachswall 22 an der gleichen Stelle angebracht.

Für die Zahnaufstellung werden nun die beiden Referenzmuster virtuell in Übereinstimmung gebracht. Die Höhen- und Seitenlage der Zähne wird damit relativ zu dem Lächelbild festgelegt, und aus der Darstellung gemäß Fig. 6 ist die Einblendung dieses virtuellen Wachswalles in das Lächelbild ersichtlich.

Aus Fig. 7 ist die Anbringung des Referenzmusters 34 auf dem digital gescannten und erstellten Oberkiefer-Wachswall des Patienten ersichtlich. Die Zusammenführung erfolgt zwischen dem 2D-Lächelbild und den 3D-Scandaten der Abformung einschließlich des oder der gescannten Referenzkörper. Das kalibrierte Lächelbild des Patienten wird genutzt, um die 3D-Zahnaufstellung anhand des eingeblendeten Lächelbildes zu kontrollieren und eine sogenannte virtuelle Einprobe der neuen Prothese vorzunehmen.

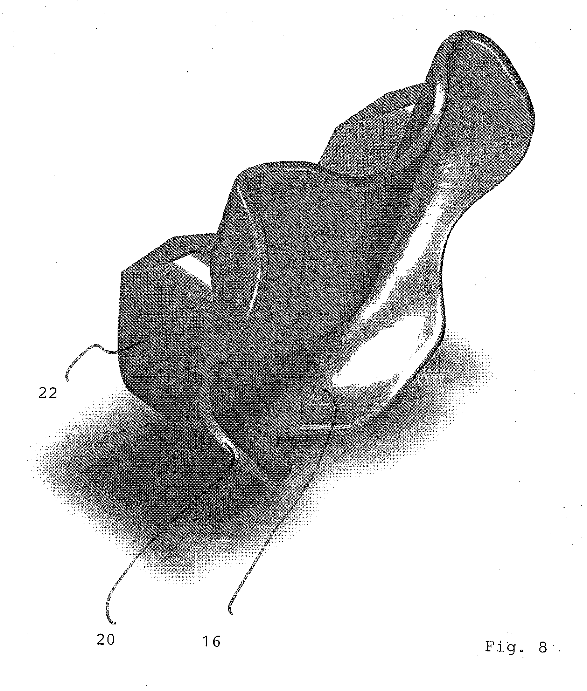

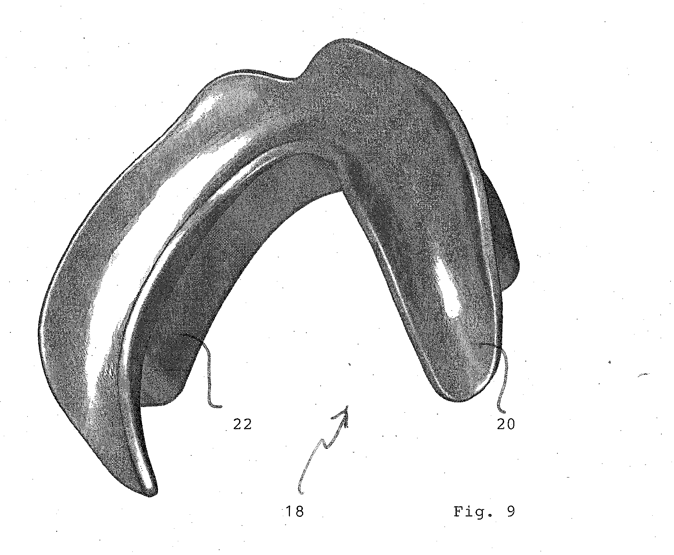

Aus Fig. 8 und 9 sind weitere Ausführungsformen von Abformlöffeln 16 und 18 ersichtlch. Die Abformlöffel bilden je einen im wesentlichen U-Kanal für die Aufnahme des Abformmaterials, so dass, rund ein Stück an die betreffenden Kieferkammbögen 20 sind die Wachswälle 22 angeformt.

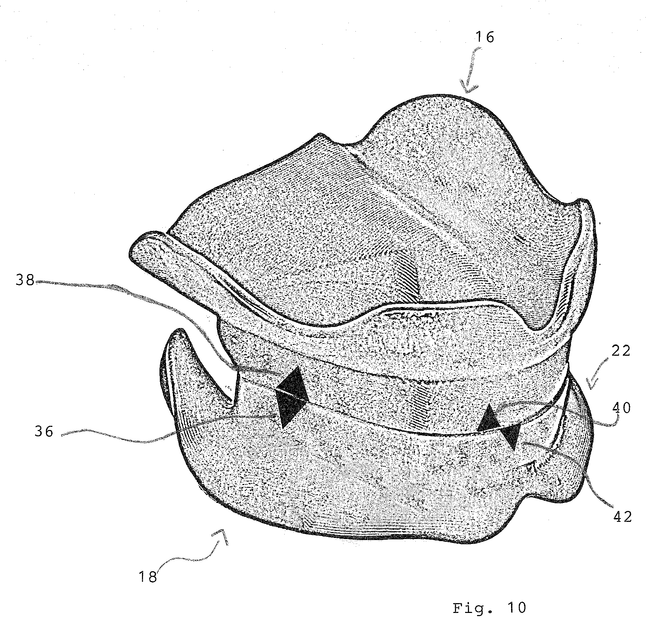

Aus Fig. 10 ist eine weitere Ausführungsform von Abformlöffeln 16 und 18 ersichtlich. Diese weisen an ihren einander zugewandten Flächen, gleichsam den Okklusionsflächen, Aussparungen 36, 38, 40, 42 auf. Diese sind in Fig. 10 zur Sichtbarmachung vestibulär dargestellt, liegen jedoch tatsächlich je in der Okklusionsfläche.

Die Aussparungen sind in an sich bekannten Weise dafür bestimmt, Verschlüsselungsmaterial oder Verschlüsselungskörper aufzunehmen, um so die Abformlöffel 16 und 18 gegeneinander zu verschlüsseln.

## Patentansprüche

1. Abformlöffel, die in mindestens zwei Standardgrößen hergestellt sind und aus einem Oberkiefer-Kieferkammbogen (20) und einem Unterkiefer-Kieferkammbogen (20) bestehen, wobei die Kieferkammbögen einander zugewandte Wachswälle tragen, die gegeneinander insbesondere verschlüsselbar sind, wobei jeder Kieferkammbogen (20) mit seinem zugehörigen Wachswall (22) einstückig ausgebildet ist und die Kieferkammbögen aus Wachs bestehen, welches bei weniger als 100 °C durch Einwirkung einer Kraft von weniger als 100 Newton, auf den distalen Endbereich jedes Kieferkammbogens (20) verformbar, insbesondere verbreiterbar und verschmälerbar ist, **dadurch gekennzeichnet, dass** die Wachswälle Aussparungen (36, 38, 40, 42) aufweisen, wobei die Oberkiefer-Aussparung (38, 40) und die Unterkiefer-Aussparung (36, 42) einander zugewandt und,
insbesondere zur Erzeugung einer Verschlüsselung, für den formschlüssigen Eingriff ineinander oder aneinander bestimmt sind, wobei in den Aussparungen (36, 38, 40, 42) ein Verschlüsselungsmaterial oder ein Verschlüsselungskörper aufnehmbar ist.

2. Abformlöffel nach Anspruch 1, **dadurch gekennzeichnet, dass** die Kieferkammbögen aus Wachs bestehen, welches bei weniger als 80 °C, und insbesondere bei mehr als 40 °C, und welches insbesondere bei weniger als 50 Newton, auf den distalen Endbereich jedes Kieferkammbogens (20) verformbar, insbesondere verbreiterbar und verschmälerbar ist.

3. Abformlöffel nach einem der Ansprüche 1 oder 2, **dadurch gekennzeichnet, dass** die Verformbarkeit mindestens auch eine plastische Verformbarkeit ist und/oder dass jeder Abformlöffel (10) bei Raumtemperatur von Hand nicht plastisch verformbar ist und bei einer gegenüber der Raumtemperatur erhöhten Temperatur, die insbesondere zwischen 40°C und 100°C liegt, von Hand plastisch verformbar ist.

4. Abformlöffel nach einem der vorhergehenden Ansprüche, **dadurch gekennzeichnet, dass** der Kieferkammbogen (20) bei Körpertemperatur bei Einwirkung einer Kraft im Bereich physiologisch wirkender Kaukräfte in oraler bzw. bukkaler Richtung plastisch unverformbar ist, also bei Einwirkung dieser Kraft sich nicht bleibend verformt.

5. Abformlöffel nach einem der vorhergehenden Ansprüche, **dadurch gekennzeichnet, dass** ein Referenzmuster (34) auf dem Wachswall (22) und/oder dem Kieferkammbogen angebracht ist und/oder als Verschlüsselungskörper ein Stützstiftregistrat vorgesehen ist, das in mindestens einen Wachswall (22) eingedrückt ist, die Kieferkammbögen (20) gegeneinander verschlüsselt.

6. Abformlöffel nach einem der vorhergehenden Ansprüche, **dadurch gekennzeichnet, dass** jeder Abformlöffel (10) eine Kieferkammnut aufweist, welche Kieferkammnut von einer oralen Nutseitenflanke und einer bukkalen Nutseitenflanke begrenzt ist und dass die Nutseitenflanken, insbesondere nach Erwärmung über die Mund-/Körpertemperatur hinaus, durch Einwirkung einer Kraft von weniger als 100 Newton in oraler oder bukkaler Richtung plastisch verformbar sind.

7. Abformlöffel nach einem der vorhergehenden Ansprüche, **dadurch gekennzeichnet, dass** die Kieferkammnut des Abformlöffels (10) für die Aufnahme eines Abformmaterials (32) bestimmt ist, das bei Mund-/Körpertemperatur plastisch verformbar ist, und dass das Abformmaterial (32) bei Mund-/Körpertemperatur nachgiebiger als das Material des Kieferkammbogens (20) und/oder des Wachswalls (22) ist.

8. Abformlöffel nach einem der vorhergehenden Ansprüche, **dadurch gekennzeichnet, dass** der Abformlöffel (10) frei von einer Bissgabel ist und in den geschlossenen Mund eines Patienten passt.

9. Abformanordnung mit
- einer Scanvorrichtung,
- einer Verarbeitungsvorrichtung, und
- einem Abformlöffel (10) nach einem der Ansprüche 1 bis 8 und einem basierend auf einem Abformergebnis erstellten Modell, wobei
die Scanvorrichtung geeignet ist, die Kieferkammbögen mit integrierten Wachswällen gemeinsam, also in verschlüsselter Form, drei-dimensional zu scannen, und
die Verarbeitungsvorrichtung geeignet ist, eine Okklusionsebene, die basierend auf den Werten eines Okklusionoms erfasst wurde, in die Scandaten einzubringen.

10. Abformanordnung nach Anspruch 9, **dadurch gekennzeichnet, dass** die Verarbeitungsvorrichtung geeignet ist, eine virtuelle Zahnaufstellung zu erstellen, sodass die Zähne in an sich bekannter Weise nach zahntechnischen Regeln, gegebenenfalls unter Beachtung der aufgebrachten Ästhetiklinien, angeordnet werden.

11. Verfahren zur Herstellung einer Prothese, insbesondere einer Vollprothese, welche Prothese über eine Abformanordnung nach Ansprüche 9 bis 10 hergestellt wird, **dadurch gekennzeichnet, dass** die verschlüsselten Kieferkammbögen mit integrierten Wachswällen gemeinsam, also in verschlüsselter Form, von der Scannvorrichtung 3-dimensional gescannt werden, und die Okklusionsebene, die basierend auf den Werten eines Okklusionoms erfasst wurde, von der Verarbeitungsvorrichtung in die Scandaten eingebracht wird.

12. Verfahren nach Anspruch 11, **dadurch gekennzeichnet, dass** anschließend hieran durch die Verarbeitungsvorrichtung eine virtuelle Zahnaufstellung erfolgt, wobei die Zähne in an sich bekannter Weise nach zahntechnischen Regeln, gegebenenfalls unter Beachtung der aufgebrachten Ästhetiklinien, angeordnet werden.

## Claims

1. Impression trays manufactured in at least two standard sizes and consisting of an upper jaw ridge arch (20) and a lower jaw ridge arch (20), wherein the alveolar ridges are provided with wax walls facing each other, which especially are encryptable in relation to each other, wherein each alveolar ridge arch (20) is integrally formed with the wax wall (22) associated thereto, and the alveolar ridge arches consist of wax which is deformable, especially widenable and narrowable, at less than 100°C by applying a force of less than 100 Newton onto the distal end region of each alveolar ridge arch (20), **characterized in that** the wax walls comprise recesses (36, 38, 40, 42), the upper jaw recess (38, 40) and the lower jaw recess (36, 42) facing each other, and especially are adapted for creating a cipher for positively engaging with or to each other, wherein a cipher material or a cipher body is receivable in the recesses (36, 38, 40, 42).

2. The impression trays according to claim 1, **characterized in that** the alveolar ridges consist of wax which is deformable, especially widenable and narrowable, on the distal end region of each alveolar ridge (20), at less than 80°C, and especially at more than 40°C, and especially at less than 50 Newton.

3. The impression trays according to one of claims 1 or 2, **characterized in that** deformability at least also represents plastic deformability and/or **in that** each impression tray (10) is not plastically deformable by hand at room temperature, and is plastically deformable by hand at a temperature higher than room temperature, especially between 40 °C and 100 °C.

4. The impression trays according to one of the preceding claims, **characterized in that** the alveolar ridge (20) is plastically non-deformable in the oral or buccal direction at a body temperature, when subjected to a force in the range of physiologically acting chewing forces, i.e. it does not permanently deform when subjected to this force.

5. The impression trays according to one of the preceding claims, **characterized in that** a reference pattern (34) is applied to the wax wall (22) and/or the alveolar ridge arch and/or a supporting pin registration is provided as an cipher body, which is pressed into at least one wax wall (22), ciphering the alveolar ridges (20) with respect to each other.

6. The impression trays according to one of the preceding claims, **characterized in that** each impression tray (10) has an alveolar ridge groove, which alveolar ridge groove is delimited by an oral groove side flank and a buccal groove side flank, and **in that** the groove side flanks are plastically deformable in the oral or buccal direction by the action of a force of less than 100 Newton, especially following heating above mouth/body temperature.

7. The impression trays according to one of the preceding claims, **characterized in that** the alveolar ridge groove of the impression tray (10) is for receiving an impression material (32) which is plastically deformable at a mouth/body temperature, and **in that** the impression material (32) is more resilient at a mouth/body temperature than the material of the alveolar ridge (20) and/or the wax wall (22).

8. The impression trays according to one of the preceding claims, **characterized in that** the impression tray (10) is without a bite fork and fits into the closed mouth of a patient.

9. An impression arrangement comprising:
- a scanning device,
- a processing device, and
- an impression tray (10) according to one of claims 1 to 8, and a model made on the basis of an impression result, wherein the scanning device is adapted for three-dimensionally scanning the alveolar ridges together with integrated wax walls, i.e. in a state of being ciphered, and
the processing device is adapted for implementing an occlusal plane, which has been detected based on the values of an occlusionoma, into the scan data.

10. The impression arrangement according to claim 9, **characterized in that** the processing device is suitable for creating a virtual teeth set-up such that the teeth are arranged in a manner known per se according to the dental rules, if necessary by taking into account the applied aesthetic lines.

11. A method for the manufacture of a prosthesis, especially a full denture, which prosthesis is manufactured using an impression arrangement according to claims 9 to 10, **characterized in that** the encoded alveolar ridges together with integrated wax walls are three-dimensionally scanned, i.e. in a state of being ciphered, using the scanning device, and the occlusal plane, which has been recorded based on the values of an occlusionoma, is implemented into the scan data by the processing device.

12. The method according to claim 11, **characterized in that** a virtual tooth set-up will subsequently be performed using the processing device, wherein the teeth are treated in a manner known per se following the dental technical rules, in accordance with the applied aesthetic lines, if required.

## Revendications

1. Porte-empreintes qui sont fabriqués en au moins deux tailles standard et qui sont constitués d'une arcade de crête alvéolaire supérieure (20) et d'une arcade de crête alvéolaire inférieure (20), où les arcades de crête alvéolaires présentent des bourrelets de cire tournées l'une vers l'autre, qui peuvent en particulier être verrouillées l'une par rapport à l'autre, où chaque arcade de crête alvéolaire (20) est formée en une seule pièce avec son bourrelet de cire (22) associée et les arcades de crête alvéolaires sont constitués de cire qui a été préparée à moins de 100°C en appliquant une force inférieure à 100 Newton, peuvent être déformées, en particulier élargies et rétrécies, sur la zone d'extrémité distale de chaque crête alvéolaire (20), **caractérisés en ce que** les crêtes de cire présentent des évidements (36, 38, 40, 42), l'évidement de la mâchoire supérieure (38, 40) et l'évidement de la mâchoire inférieure (36), 42) se faisant face et en particulier pour générer un verrouillage, sont destinés à un engagement positif l'un dans l'autre ou l'un contre l'autre, où un matériau de verrouillage ou un corps de verrouillage peut être logé dans les évidements (36, 38, 40, 42).

2. Porte-empreintes selon la revendication 1, **caractérisés en ce que** les arcades de crête alvéolaires sont constituées de cire qui peut être déformée, en particulier élargie et rétrécie sur la région de l'extrémité distale de chaque arcade crête alvéolaire (20), à moins de 80°C, et en particulier à plus de 40°C et en particulier à moins de 50 Newton.

3. Porte-empreintes selon l'une des revendications 1 ou 2, **caractérisé en ce que** la déformabilité est au moins aussi une déformabilité plastique et/ou que chaque porte-empreinte (10) n'est pas plastiquement déformable à la main à température ambiante et est plastiquement déformable à la main à une température élevée par rapport à la température ambiante, qui se situe en particulier entre 40°C et 100°C.

4. Porte-empreintes selon l'une des revendications précédentes, **caractérisé en ce qu'**à la température du corps sous l'action d'une force dans la gamme des forces de mastication à action physiologique, l'arcade de crête alvéolaire (20) est plastiquement indéformable dans le sens oral ou buccal c'est-à-dire qu'elle ne se déforme pas de façon permanente lors de l'application de cette force.

5. Porte-empreintes selon l'une des revendications précédentes, **caractérisé en ce qu'**un modèle de référence (34) est appliqué sur le bourrelet de cire (22) et/ou l'arcade de la crête alvéolaire et/ou qu'un registre de broches de support est prévu comme corps de verrouillage, qui est pressé dans au moins un bourrelet de cire (22), verrouille les arcades de crête alvéolaires (20) les unes contre les autres.

6. Porte-empreinte selon l'une des revendications précédentes, **caractérisé en ce que** chaque porte-empreinte (10) présente une rainure de crête de mâchoire, où ladite rainure de crête de mâchoire est délimitée par un flanc latéral de rainure buccale et un flanc latéral de rainure orale, et **en ce que** les flancs latéraux de rainure sont plastiquement déformables dans la direction orale ou buccale par l'action d'une force inférieure à 100 Newton, en particulier après un échauffement au-dessus de la température buccale/corporelle.

7. Porte-empreinte selon l'une des revendications précédentes, **caractérisé en ce que** la rainure de crête de mâchoire du porte-empreinte (10) est conçue pour recevoir un matériau d'impression (32) qui est plastiquement déformable à la température buccale/corporelle, et **en ce que** le matériau d'impression (32) est plus élastique à la température buccale/corporelle que le matériau de l'arcade de la crête de la mâchoire (20) et/ou du bourrelet de cire (22).

8. Porte-empreinte selon l'une des revendications précédentes, **caractérisé en ce que** le porte-empreinte (10) est exempt de fourchette d'enregistrement et rentre dans la bouche fermée d'un patient.

9. Arrangement d'empreinte avec
- un dispositif de balayage,
- un dispositif de traitement, et
- un porte-empreinte (10) selon l'une quelconque des revendications 1 à 8 et un modèle préparé sur la base d'un résultat d'impression, où le dispositif de balayage est adapté pour le balayage tridimensionnel commun des arcs de la crête alvéolaire avec des bourrelets de cire intégrées, c'est-à-dire sous forme verrouillée, et le dispositif de traitement est adapté pour introduire dans les données du balayage un plan occlusal, qui a été acquis sur la base des valeurs d'un occlusionome.

10. Disposition d'empreinte selon la revendication 9, **caractérisée en ce que** le dispositif de traitement est adapté pour produire une disposition virtuelle des dents, de telle manière que les dents sont disposées d'une manière connue en soi selon les règles dentaires, en tenant compte le cas échéant des lignes esthétiques appliquées.

11. Procédé de fabrication d'une prothèse, en particulier d'une prothèse complète, qui est fabriquée au moyen d'un dispositif d'empreinte selon les revendications 9 à 10, **caractérisée en ce que** les arcades de crête alvéolaires codées avec des bourrelets de cire intégrées sont balayées ensemble en trois dimensions, c'est-à-dire sous forme codée, par le dispositif de balayage, et le plan d'occlusion, qui a été enregistré sur la base des valeurs d'un occlusionome, est introduit dans les données de balayage par le dispositif de traitement.

12. Procédé selon la revendication 11, **caractérisé en ce qu'**à continuation, un montage virtuel des dents est effectué par le dispositif de traitement, où les dents sont disposées d'une manière connue en soi selon les règles de la technique dentaire, en tenant compte le cas échéant des lignes esthétiques appliquées.
